# EUROPÄISCHE PATENTSCHRIFT

(11) **EP 1 837 379 B1**
(45) Veröffentlichungstag und Bekanntmachung des Hinweises auf die Patenterteilung: **07.11.2018**
(21) Anmeldenummer: 07005044.8
(22) Anmeldetag: 12.03.2007
(51) Int. Cl.: C09C 1/00, A61K 8/25, A61Q 1/08, C03B 37/005, A61Q 19/00, A61K 8/02, C03C 4/02, C03C 12/00, A61Q 3/02, A61Q 5/12, A61Q 19/08, A61Q 1/02, A61Q 1/04, A61Q 1/10, A61Q 1/12, A61Q 5/02, A61Q 5/06, A61Q 17/04, A61Q 19/04, A61Q 19/10

(54) **Glasplättchen und deren Verwendung als transparenter Füllstoff**
Glass platelets and their application as transparent fillers
Petites pastilles de verre et leur utilisation en tant qu'agent de remplissage transparent

(30) Priorität: 24.03.2006 DE 10614095
(43) Veröffentlichungstag der Anmeldung: 26.09.2007
(60) Teilanmeldung: 10011365.3
(73) Patentinhaber: Merck Patent GmbH, 64293 Darmstadt (DE)
(72) Erfinder: Schulz, Elke, Dr., 64390 Erzhausen (DE); Hochstein, Veronika, 76646 Bruchsal (DE)

(56) Entgegenhaltungen:
- EP-A- 1 602 632
- EP-A1- 0 289 240
- EP-A1- 2 008 641
- EP-A2- 1 469 042
- EP-B1- 0 912 640
- WO-A-2005/063637
- WO-A1-97/46624
- WO-A1-2005/063637
- WO-A1-2006/018196
- WO-A1-2007/054379
- WO-A2-03/006558
- DE-A1- 10 259 301
- US-A- 2 863 783
- US-A- 4 363 889
- US-A1- 2002 011 186
- US-A1- 2004 052 743
- US-A1- 2004 123 778
- US-A1- 2004 134 385
- US-A1- 2004 177 788
- US-A1- 2004 221 770
- US-A1- 2005 118 122
- US-A1- 2005 176 850

## Beschreibung

Die vorliegende Erfindung betrifft die Verwendung von Glasplättchen, insbesondere als transparenter Füllstoff in kosmetischen Formulierungen. Glasplättchen mit bestimmten Dimensionen sind aufgrund ihrer Transparenz als Füllstoff in kosmetischen Formulierungen geeignet, da sie nicht die Grundfarbe der Formulierung verändern und gleichzeitig das Hautgefühl verbessern. Eingefärbte Glasplättchen zielen dagegen darauf ab die Grundfarbe der Formulierung zu verändern.

Kosmetische Formulierungen, wie z.B. Puder, Make-ups, enthalten in der Regel organische und/oder anorganische Füllstoffe. Bei den Füllstoffen handelt es sich um partikuläre Stoffe, die keinen Farbeffekt im Produkt, d. h. in der kosmetischen und dermatologischen Zubereitung selbst oder auf der Haut hervorrufen. In der Kosmetik werden beispielsweise Füllstoffe aus der Gruppe Polymethylmethacrylat, Methylmethacrylat Cross-Polymer, Glimmer, Nylon-Pulver, reine oder gefüllte Melaminharze, Talkum, SiO₂, Kaolin, Oxide oder Hydroxide von Aluminium, Magnesium, Calcium, Zink, BiOCI, Bariumsulfat, Calciumsulfat, Calciumcarbonat, basische Erdalkalicarbonate, wie z.B. Calcium- oder Magnesiumcarbonat, Kohlenstoff, sowie physikalische oder chemische Kombinationen dieser Stoffe eingesetzt. Bezüglich der Partikelform des Füllstoffes gibt es keine Einschränkungen. Sie kann den Anforderungen gemäß unregelmäßig, plättchenförmig, sphärisch oder nadelförmig sein. Die kommerziell erhältlichen Füllstoffe zeigen dabei häufig den Nachteil, dass sie eine Eigenfarbe aufweisen, die in der Regel nicht gewünscht ist.

Der Erfindung liegt die Aufgabe zugrunde Füllstoffe, insbesondere für kosmetische Formulierungen zu finden, die es erlauben Formulierungen herzustellen, die die Grundfarbe nicht beeinträchtigen bzw. gezielt verändern.

Überraschenderweise wurde nun gefunden, dass bei Verwendung amorpher Glasplättchen mit genau definierten Dimensionen diese Aufgabe erfüllt wird.

Glasplättchen sind beispielsweise bekannt aus der US 4,363,889 A, EP 1 602 632 A, EP 2 008 641 A, WO 2005/063637 A, US 2,863,783 A, US 2004/191 198 A und WO 01/82883 A. WO 2004/056716 A beschreibt ein spezielles Herstellungsverfahren von Glasplättchen, wobei nach einer Erklärung des Erfinders der WO 2004/056716 A, die er im Jahre 2013 im Prüfungsverfahren der EP 2 203 394 B1 eingereicht hat, dieses spezielle Verfahren doch nicht zur Herstellung von Glasplättchen mit einer Dicke ≤ 250 nm geeignet sein soll.

Gegenstand der Erfindung ist gemäß Anspruch 1 die Verwendung von transparenten Glasplättchen, die sich dadurch auszeichnen, dass sie eine Dicke von < 1 µm und eine mittlere Teilchengröße von 1-150 µm aufweisen, in Emulsionen, Zahnpasta, wasserfreien auf Öl-/Wachsbasierenden Produkten und Puderprodukten.

Die Glasplättchen finden insbesondere als Füllstoff in der dekorativen und pflegenden Kosmetik Verwendung. Sie können aber auch in allen Formulierungen eingesetzt werden, wo üblicherweise Füllstoffe eingesetzt werden, wie beispielsweise in Farben, Lacken und Kunststoffen.

Im Gegensatz zum Stand der Technik, wo kosmetische Formulierungen enthaltend Glaspulver mit unregelmäßig geformten Teilchen beschrieben werden, zeigen die Glasplättchen gemäß Anspruch 1 ein besseres Füllstoffverhalten und ein sehr gutes Hautgefühl.

Unter Glas wird hier ein anorganisches Stoffgemisch verstanden, das aus dem geschmolzenen Zustand ohne Kristallisation abgekühlt und einen erstarrten Zustand angenommen hat. Die verwendeten Glasplättchen sind amorph.

Geeignete Gläser sind alle dem Fachmann bekannten Gläser, wie in Anspruch 1 angegeben, beispielsweise Silikatgläser, wie Kalknatronglas, Borosilikatglas, Aluminosilikatglas, Bleikristallglas, E-, A-, C-, ECR-Glas, Duranglas, Fensterglas, Laborglas, etc. Derartige Gläser werden aus Sand, Kalk, Tonerde, Borverbindungen, Pottasche, Soda, usw. erschmolzen und in einem geformten Zustand erstarren gelassen. Die verwendeten Glasplättchen bestehen vorzugsweise aus C-, E-, ECR- oder Borosilikatglas. Es können selbstverständlich auch Gemische von verschiedenen Glasplättchen eingesetzt werden, die sich nur in der Glaszusammensetzung unterscheiden.

Durch den Zusatz von anorganischen Farbmitteln können die Glasplättchen bei der Herstellung gezielt eingefärbt werden. Geeignete

Farbmittel sind solche, die bei der Schmelztemperatur des Glases sich nicht zersetzen. Das Farbmittel wird in der Regel in Mengen von 0,1 - 50

Gew. %, insbesondere von 0,2 - 25 Gew.% und ganz besonders bevorzugt von 0,5 -10 Gew. %, der Glasschmelze zugesetzt.

Geeignete Färbemittel sind insbesondere die Kationen oder komplexe Anionen der Elemente Cu, Cr, Mn, Fe und Co und/oder deren Kombinationen. Durch den Zusatz der Ionen können intensive Blau-, Grün-, Gelb-, Orange- oder Rotfärbungen erhalten werden. Geeignete Färbemittel sind weiterhin TiO₂ oder elementare Edelmetalle.

Die Glasplättchen besitzen eine Dicke von < 1 µm, vorzugsweise von 100 nm -1 µm, insbesondere von 150 - 800 nm und ganz besonders bevorzugt von 200 - 600 nm. Die mittlere Teilchengröße beträgt 1 - 150 µm, insbesondere 10 -100 µm und ganz besonders bevorzugt 5 - 35 µm.

Die verwendeten Glasplättchen besitzen vorzugsweise einen Formfaktor (aspect ratio: Durchmesser / Dicke-Verhältnis) von 1 - 1500, insbesondere von 10 - 700, und ganz besonders bevorzugt von 50 - 200.

Die Glasplättchen zeichnen sich weiterhin durch eine Brechzahl von 1,2 - 2,1, insbesondere von 1,3 -1,9 und ganz besonders bevorzugt von 1,4 - 1,6, aus.

Die Glasplättchen sind amorph und weisen eine Transparenz von ≥ 90 %, insbesondere ≥ 93 %, und ganz besonders bevorzugt von ≥ 95 % auf (Messung der Transmission im Spektralbereich von 400 - 700 nm mit UV-VIS-IR-Spektrometer: Typ Perkin Elmer Lambda 900 mit Ulbrichtkugel (150 mm Durchmesser)).

Die Glasplättchen haben vorzugsweise eine Ölzahl (bestimmt nach DIN EN ISO 787-5: 1995-10) im Bereich von 20 bis 130, insbesondere von 30 bis 110, ganz besonders bevorzugt von 50 bis 90.

Die Glasplättchen werden aus der Schmelze heraus mit den bekannten Verfahren, wie z.B. Rohreblasen (Nippon Sheet Glas), Schleuderverfahren (Glassflake Ltd.) hergestellt. Besonders bevorzugt werden die Glasplättchen nach dem Schleuderverfahren hergestellt, wie es beispielsweise in der EP 0 289 240 oder WO 2005/063637 beschrieben wird.

Gegenstand der Erfindung ist insbesondere die Verwendung von optisch klaren ungefärbten und in massegefärbten Glasplättchen mit Dicken von < 1 µm und einer mittleren Teilchengröße von 1 - 150 µm.

Die Glasplättchen werden insbesondere als Füllstoffe eingesetzt, vorzugsweise in der pflegenden und dekorativen Kosmetik, da die transparenten Glasplättchen in der Formulierung unsichtbar sind und damit die Eigenfarbe der Formulierung nicht verfälschen.

Aufgrund der variierbaren Dicken und Teilchengrößen lassen sich in den kosmetischen Formulierungen gezielt Glitzereffekte einstellen, die unter direkter Lichteinstrahlung sehr gut zu sehen sind. Mit Hilfe von sehr dünnen Glasplättchen, vorzugsweise mit Dicken von < 500 nm und/oder sehr feinen Fraktionen mit Teilchengrößen von < 50 µm ist es aber auch möglich einen Mattierungseffekt einzustellen, der den unerwünschten Glanz, der z.B. bei Gesichtspudern benötigt wird, zu unterdrücken. Ein weiterer Effekt ist, dass der Hautglanz reduziert wird. Weiterhin zeichnen sich die Glasplättchen durch ihr sehr gutes Hautgefühl aus. Der Einsatz der Glasplättchen führt weiterhin zu einer Verbesserung der Applikationseigenschaften der Formulierung sowie der Textur des Produktes, und man erhält im Vergleich zu den im Markt befindlichen Füllstoffen reinere Farben der Formulierung.

Die Konzentration der Glasplättchen im zu pigmentierenden Anwendungssystem liegt in der Regel zwischen 0,01 und 95 Gew.%, vorzugsweise zwischen 0,1 und 50 Gew.% und insbesondere zwischen 1,0 und 10 Gew.%, bezogen auf den Gesamtfestkörpergehalt des Systems. Sie ist in der Regel abhängig vom konkreten Anwendungs-fall und kann bei losen Pudern bis zu 95 Gew. % betragen.

### Vorzugsweise enthalten

- Emulsionen 0,1 - 30 Gew.%, insbesondere 1 - 15 Gew.%,
- pigmenthaltige Emulsionen 0,1 - 50 Gew.%, insbesondere 1 - 15 Gew.% in Abhängigkeit von der Textur,
- Zahnpasta 0,1 - 60 Gew.%, insbesondere 1 - 50 Gew.%,
- wasserfreie auf Öl-/Wachsbasierende Produkte 0,1 - 75 Gew.%, insbesondere 0,5 - 65 Gew.%,
- Puderprodukte 0,1 - 95 Gew.%, insbesondere 1 - 75 Gew.%,
an Glasplättchen, bezogen auf die Gesamtformulierung.

Die Glasplättchen sind einfach und leicht zu handhaben, da sie sich sehr leicht in eine Formulierung einarbeiten lassen. Die Glasplättchen können allein oder im Gemisch mit weiteren kosmetischen Hilfs-, Träger- und Wirkstoffen durch einfaches Einrühren in das Anwendungssystem eingearbeitet werden. Ein aufwendiges Dispergieren der Glasplättchen ist nicht erforderlich.

Selbstverständlich können die Glasplättchen in den Formulierungen auch mit jeder Art von kosmetischen Roh- und Hilfsstoffen sowie Wirkstoffen kombiniert werden. Dazu gehören u.a. Wasser, Alkohole, Polyole, polare und unpolare Öle, Fette, Wachse, Filmbildner, Polymere, Co-Polymere, Tenside, Radikalfänger, Antioxidantien, wie z.B. Vitamin C oder Vitamin E, Stabilisatoren, Geruchsverstärker, Silikonöle, Emulgatoren, Duftstoffe, Lösemittel wie z.B. Ethanol, Ethylacetat oder Butylacetat, Konservierungsmittel und allgemein anwendungstechnische Eigenschaften bestimmende Hilfsstoffe, wie z.B. Verdicker und rheologische Zusatzstoffe wie etwa Bentonite, Hektorite, Siliciumdioxide, Ca-Silicate, Gelatine, hochmolekulare Kohlenhydrate und/oder oberflächenaktive Hilfsmittel, etc.

Geeignete Wirkstoffe sind z.B. Insect Repellents, anorganische UV-Filter, wie z.B. TiO₂, UV A/BC-Schutzfilter (z.B. OMC, B3, MBC), auch in verkapselter Form, Anti-Ageing-Wirkstoffe, Vitamine und deren Derivate (z.B. Vitamin A, C, E, etc.), Selbstbräuner (z.B. DHA, Erytrolyse, u.a.) sowie weitere kosmetische Wirkstoffe, wie z.B. Bisabolol, LPO, VTA, Ectoin, Emblica, Allantoin, Bioflavanoide und deren Derivate.

Organische UV-Filter werden in der Regel in einer Menge von 0,5 bis 10 Gew.%, vorzugsweise 1 bis 8 Gew.%, anorganische Filter von 0,1 bis 30 Gew.% in kosmetische Formulierungen eingearbeitet.

Die erfindungsgemäßen Zubereitungen können darüber hinaus weitere übliche hautschonende oder hautpflegende Wirkstoffe enthalten. Dies können prinzipiell alle dem Fachmann bekannten Wirkstoffe sein. Besonders bevorzugte Wirkstoffe sind Pyrimidincarbonsäuren und/oder Aryloxime.

Unter den kosmetischen Anwendungen ist insbesondere die Verwendung von Ectoin und Ectoin-Derivaten zur Pflege von gealterter, trockener oder gereizter Haut zu nennen. So wird in der europäischen Patentanmeldung EP-A-0 671 161 insbesondere beschrieben, dass Ectoin und Hydroxyectoin in kosmetischen Zubereitungen wie Pudern, Seifen, tensidhaltigen Reinigungsprodukten, Lippenstiften, Rouge, Make-Up, Pflegecremes und Sonnenschutzpräparaten eingesetzt werden.

Bei Selbstbräunungscremes, -lotionen, -sprays, etc. enthaltend beispielsweise den Selbstbräuner DHA (Dihydroxyaceton) und ein Effektpigment mit abschließender TiO₂-Schicht, wird das DHA langsam in der Formulierung abgebaut. Bei Verwendung der Glasplättchen, anstelle der TiO₂-beschichteten Effektpigmente, in der Formulierung bleibt das DHA in seiner Wirkung voll erhalten.

Es versteht sich von selbst, dass für die verschiedenen Anwendungszwecke die Glasplättchen auch vorteilhaft in Abmischung mit Effektpigmenten, wie z.B. Perlglanzpigmenten, Interferenzpigmenten, goniochromatischen Pigmenten, BiOCI-Plättchen, Mehrschichtpigmenten, Metallpigmenten, organischen Farbstoffen, organischen Farbpigmenten und anderen Pigmenten, wie z.B. transparenten und deckenden Weiß-, Bunt- und Schwarzpigmenten sowie mit plättchenförmigen Eisenoxiden, holographischen Pigmenten, LCPs (Liquid Crystal Polymers) und herkömmlichen transparenten, bunten und schwarzen Glanzpigmenten auf der Basis von metalloxidbeschichteten Glimmer- und SiO₂-Plättchen, etc., verwendet werden können. Die Glasplättchen können in jedem Verhältnis mit handelsüblichen (Effekt-)Pigmenten gemischt werden. Das Gewichtsverhältnis Glasplättchen zu Pigment kann je nach Farbausprägung 1 : 99 zu 99 : 1 sein. Bei farbigen Texturen ist der Pigmentanteil höher als bei weniger farbigen.

Geeignete Farbmittel in der Kosmetik sind insbesondere Effektpigmente, wie z.B. Perlglanzpigmente, einschließlich Mehrschichtpigmente oder Interferenzpigmente. Als Perlglanzpigmente werden Pigmente auf der Basis plättchenförmiger, transparenter oder semitransparenter Substrate aus z.B. Schichtsilikaten, wie etwa natürlichem oder synthetischem Glimmer, Talkum, Sericit, Kaolin oder anderen silikatischen Materialien verwendet, die mit farbigen oder farblosen Metalloxiden wie z.B. TiO₂, Titansuboxide, Titanoxinitride, Fe₂O₃, Fe₃O₄, FeOOH, SnO₂, Cr₂O₃, ZnO, CuO, NiO und anderen Metalloxiden allein oder in Mischung in einer einheitlichen Schicht oder in aufeinanderfolgenden Schichten beschichtet sind.

Perlglanzpigmente sind z. B. aus den deutschen Patenten und Patentanmeldungen 14 67 468, 19 59 998, 20 09 566, 22 14 454, 22 15 191, 22 44 298, 23 13 331, 25 22 572, 31 37 808, 31 37 809, 31 51 343, 31 51 354, 31 51 355, 32 11 602, 32 35 017 und P 38 42 330 bekannt und im Handel erhältlich, z.B. unter den Marken Iriodin®, Timiron®, Xirona®, Colorona®, Dichrona® der Firma Merck KGaA, Darmstadt, Deutschland und/oder Rona, USA. Besonders bevorzugte Pigmentpräparationen enthalten TiO₂/Glimmer-, Fe₂O₃/Glimmer- und/oder TiO₂/Fe₂O₃-Glimmerpigmente. Die Perlglanzpigmente können zusätzlich noch eine Schicht aus Berliner Blau oder Carminrot auf der Oberfläche aufweisen.

Weiterhin bevorzugt sind beschichtete oder unbeschichtete BiOCI-Pigmente, mit TiO₂ und/oder Fe₂O₃ beschichtete SiO₂-, Glas- oder Al₂O₃-Plättchen. Die Beschichtung der SiO₂-Plättchen mit ein oder mehreren Metalloxiden kann z.B. erfolgen wie in der WO 93/08237 (naß-chemische Beschichtung) oder DE-OS 196 14 637 (CVD-Verfahren) beschrieben.

Die beispielsweise aus den deutschen Offenlegungsschriften
DE 196 18 563, DE 196 18 566, DE 196 18 569, DE 197 07 805,
DE 197 07 806, DE 197 46 067 bekannten Mehrschichtpigmente basieren auf einer plättchenförmigen, transparenten, farbigen oder farblosen Matrix, bestehend aus Glimmer (synthetisch oder natürlich), SiO₂-Plättchen, Glasplättchen, Al₂O₃-Plättchen, Polymerplättchen, und besitzen in der Regel eine Dicke zwischen 0,3 und 5 µm, insbesondere zwischen 0,4 und 2,0 µm. Die Ausdehnung in den beiden anderen Dimensionen beträgt üblicherweise zwischen 1 und 250 µm, vorzugsweise zwischen 2 und 100 µm, und insbesondere zwischen 5 und 40 µm. Die Mehrschichtpigmente bestehen aus der Matrix (Substrat) beschichtet mit Metalloxiden (mindestens 2). Die Beschichtung der Substratplättchen Glimmer, SiO₂-Plättchen, Glasplättchen, Al₂O₃-Plättchen mit mehreren Schichten erfolgt so, dass ein Schichtaufbau vorzugsweise bestehend aus alternierenden hoch- und niedrigbrechenden Schichten entsteht. Vorzugsweise enthalten die Mehrschichtpigmente 2, 3, 4, 5, 6 oder 7 Schichten, insbesondere 3, 4 oder 5 Schichten. Geeignete hochbrechende Metalloxide sind beispielsweise Titandioxid, Zirkonoxid, Zinkoxid, Eisenoxide, Eisen-TitanOxide (Eisentitanate) und/oder Chromoxid, insbesondere TiO₂ und/oder Fe₂O₃. Als niedrigbrechende Oxide kommen SiO₂ und Al₂O₃ zum Einsatz. Es kann hierfür jedoch auch MgF₂ oder ein organisches Polymer (z.B. Acrylat) eingesetzt werden. Die Beschichtung der Substratplättchen kann z.B. erfolgen wie in der WO 93/08237 (nasschemische Beschichtung) oder DE-OS-196 14 637 (CVD-Verfahren) beschrieben. Beschichtung bedeutet, dass das Substrat komplett mit einer oder mehreren Schichten umhüllt ist.

Besonders bevorzugte Mehrschichtpigmente auf Basis von Glimmer (natürlich oder synthetisch), Glasplättchen, Al₂O₃-Plättchen, Fe₂O₃-Plättchen, SiO₂-Plättchen enthalten eine Schichtenfolge TiO₂ - SiO₂ - TiO₂.

Bei den Interferenzpigmenten handelt es sich vorzugsweise um Pigmente auf der Basis von natürlichem und synthetischem Glimmer, Glasplättchen, SiO₂-Plättchen, Al₂O₃-Plättchen, die mit farbigen oder farblosen Metalloxiden wie z.B. TiO₂, Titansuboxide, Titanoxinitride, Fe₂O₃, Fe₃O₄, SnO₂, Cr₂O₃, ZnO, CuO, NiO und anderen Metalloxiden allein oder in Mischung in einer einheitlichen Schicht oder in aufeinanderfolgenden Schichten beschichtet sind.

Als plättchenförmige Farbmittel kommen vor allem Perlglanzpigmente insbesondere auf Basis von natürlichem oder synthetischem Glimmer, SiO₂-Plättchen, Fe₂O₃-Plättchen, Glasplättchen oder Al₂O₃-Plättchen, die nur mit einer Metalloxidschicht umhüllt sind, Metalleffektpigmente (Al-Plättchen, Bronzen), optisch variable Pigmente (OVP's), Flüssigkristallpolymerpigmente (LCP's) oder holographische Pigmente in Frage.

Zu den sphärischen Farbmitteln zählen insbesondere TiO₂, eingefärbtes SiO₂, CaSO₄, Eisenoxide, Chromoxide, Ruß, organische Farbpigmente, wie z.B. Anthrachinon-Pigmente, Chinacridon-Pigmente, Diketopyrrolopyrrol-Pigmente, Phthalocyanin-Pigmente, Azopigmente, Isoindolin-Pigmente. Bei den nadelförmigen Pigmenten handelt es sich vorzugsweise um BiOCI, eingefärbte Glasfasern, α-FeOOH, organische Farbpigmente, wie z.B. Azopigmente, β-Phthalocyanin CI Blue 15,3, Cromophtalgelb 8GN (Ciba-Geigy), Irgalith Blau PD56 (Ciba-Geigy), Azomethinkupferkomplex CI Yellow 129, Irgazingelb 5GT (Ciba-Geigy).

Geeignete organische Farbpigmente und Farbstoffe sind natürlichen oder synthetischen Ursprungs, wie z.B. Chromoxid, Ultramarin.

Die Glasplättchen, die vorzugsweise als Füllstoffe in kosmetischen Formulierungen ihren Einsatz finden, können selbstverständlich auch mit anderen bekannten Füllstoffen gemischt werden bzw. eingesetzt werden. Als Füllstoffe sind z. B. zu nennen synthetische organische Polymere, Polymethylmethacrylat, Methylmethacrylat Cross-Polymer, natürlicher und synthetischer Glimmer, Nylon-Pulver, reine oder gefüllte Melaminharze, Talkum, SiO₂, Glaspulver, Glaskugeln, Kaolin, Oxide oder Hydroxide von Aluminium, Magnesium, Calcium, Zink, BiOCI, Bariumsulfat, Calciumsulfat, Calciumcarbonat, Magnesiumcarbonat, basische Erdalkalicarbonate, wie z.B. Calcium- oder Magnesiumcarbonat, Kohlenstoff, sowie physikalische oder chemische Kombinationen dieser Stoffe.

Bezüglich der Partikelform der weiteren Füllstoffe gibt es keine Einschränkungen. Sie kann den Anforderungen gemäß z. B. unregelmäßig, plättchenförmig, sphärisch oder nadelförmig sein.

Ebenso können nanoskalige Dielektrika zur Verbesserung des Hautgefühls beigemischt werden. Beispiele für derartige Beimischungen sind Al₂O₃, SiO₂, ZnO oder TiO₂, die üblicherweise in Mengen von 0,01 -15 Gew.% der Formulierung zugegeben werden.

Die die Glasplättchen enthaltenden Formulierungen können dem lipophilen, hydrophilen oder hydrophoben Typ angehören. Bei heterogenen Formulierungen mit diskreten wässrigen und nicht-wässrigen Phasen können die erfindungsgemäßen Glasplättchen in jeweils nur einer der beiden Phasen enthalten oder auch über beide Phasen verteilt sein.

Die pH-Werte der Formulierungen können zwischen 1 und 14, bevorzugt zwischen 2 und 11 und besonders bevorzugt zwischen 5 und 8 liegen.

Als Anwendungsform der kosmetischen Formulierungen seien z.B. genannt Lösungen, Suspensionen, Emulsionen, PIT-Emulsionen, Pasten, Salben, Gele, Cremes, Lotionen, Puder, Seifen, tensidhaltige Reinigungspräparate, Öle, Aerosole und Sprays. Weitere Anwendungsformen sind z.B. Sticks, Shampoos und Duschbäder. Der Zubereitung können neben den erfindungsgemäßen Glasplättchen beliebige übliche Trägerstoffe, Hilfsstoffe und gegebenenfalls weitere Wirkstoffe zugesetzt werden.

Salben, Pasten, Cremes und Gele können die üblichen Trägerstoffe enthalten, z.B. tierische und pflanzliche Fette, Wachse, Paraffine, Stärke, Traganth, Cellulosederivate, Polyethylenglykole, Silicone, Bentonite, Kieselsäure, Talkum und Zinkoxid oder Gemische dieser Stoffe.
Puder und Sprays können die üblichen Trägerstoffe enthalten, z.B. Milchzucker, Talkum, Kieselsäure, Aluminiumhydroxid, Calciumsilikat und Polyamid-Pulver oder Gemische dieser Stoffe. Sprays können zusätzlich die üblichen Treibmittel, z.B. Chlorfluorkohlenwasserstoffe, Propan/Butan oder Dimethylether, enthalten.

Lösungen und Emulsionen können die üblichen Trägerstoffe wie Lösungsmittel, Lösungsvermittler und Emulgatoren, z.B. Wasser, Ethanol, Isopropanol, Ethylcarbonat, Ethlyacetat, Benzylalkohol, Benzylbenzoat, Propylenglykol, 1,3-Butylglykol, Öle, insbesondere Baumwollsaatöl, Erdnussöl, Maiskeimöl, Olivenöl, Rizinusöl und Sesamöl, Glycerinfettsäureester, Polyethylenglykole und Fettsäureester des Sorbitans oder Gemische dieser Stoffe enthalten.

Suspensionen können die üblichen Trägerstoffe wie flüssige Verdünnungsmittel, z.B. Wasser, Ethanol oder Propylenglykol, Suspendiermittel, z.B. ethoxylierte Isostearylalkohole, Polyoxyethylensorbitester und Polyoxyethylensorbitanester, mikrokristalline Cellulose, Aluminiummetahydroxid, Bentonit, Agar-Agar und Traganth oder Gemische dieser Stoffe enthalten.

Seifen können die üblichen Trägerstoffe wie Alkalisalze von Fettsäuren, Salze von Fettsäurehalbestern, Fettsäureeiweißhydrolysaten, Isothionate, Lanolin, Fettalkohol, Pflanzenöle, Pflanzenextrakte, Glycerin, Zucker oder Gemische dieser Stoffe enthalten.

Tensidhaltige Reinigungsprodukte können die üblichen Trägerstoffe wie Salze von Fettalkoholsulfaten, Fettalkoholethersulfaten, Sulfobernsteinsäurehalbestern, Fettsäureeiweißhydrolysaten, Isothionate, Imidazoliniumderivate, Methyltaurate, Sarkosinate, Fettsäureamidethersulfate, Alkylamidobetaine, Fettalkohole, Fettsäureglyceride, Fettsäurediethanolamide, pflanzliche und synthetische Öle, Lanolinderivate, ethoxylierte Glycerinfettsäureester oder Gemische dieser Stoffe enthalten.

Gesichts- und Körperöle können die üblichen Trägerstoffe wie synthetische Öle wie z.B. Fettsäureester, Fettalkohole, Silikonöle, natürliche Öle wie Pflanzenöle und ölige Pflanzenauszüge, Paraffinöle, Lanolinöle oder Gemische dieser Stoffe enthalten.

Die kosmetischen Zubereitungen können in verschiedenen Formen vorliegen. So können sie z. B. eine Lösung, eine wasserfreie Zubereitung, eine Emulsion oder Mikroemulsion vom Typ Wasser-in-ÖI (W/O) oder vom Typ ÖI-in-Wasser (O/W), eine multiple Emulsion, beispielsweise vom Typ Waser-in-ÖI-in-Wasser (W/O/W), ein Gel, einen festen Stift, eine Salbe oder auch ein Aerosol darstellen. Es ist auch vorteilhaft, Ectoine in verkapselter Form darzureichen, z. B. in Kollagenmatrices und anderen üblichen Verkapselungsmaterialien, z. B. als Celluloseverkapselungen, in Gelatine, Wachsmatrices oder liposomal verkapselt. Insbesondere Wachsmatrices wie sie in der DE-OS 43 08 282 beschrieben werden, haben sich als günstig herausgestellt. Bevorzugt werden Emulsionen. O/W-Emulsionen werden besonders bevorzugt. Emulsionen, W/O-Emulsionen und O/W-Emulsionen sind in üblicher Weise erhältlich.

Weitere Ausführungsformen stellen ölige Lotionen auf Basis von natürlichen oder synthetischen Ölen und Wachsen, Lanolin, Fettsäureestern, insbesondere Triglyceriden von Fettsäuren, oder ölig-alkoholische Lotionen auf Basis eines Niedrigalkohols, wie Ethanol, oder eines Glycerols, wie Propylenglykol, und/oder eines Polyols, wie Glycerin, und Ölen, Wachsen und Fettsäureestern, wie Triglyceriden von Fettsäuren, dar.

Feste Stifte bestehen aus natürlichen oder synthetischen Wachsen und Ölen, Fettalkoholen, Fettsäuren, Fettsäureestern, Lanolin und anderen Fettkörpern.

Bei den kosmetischen Ölen handelt es sich vorzugsweise um Mineralöl, hydriertes Polyisobuten, synthetisches oder aus Naturprodukten hergestelltes Squalan, kosmetische Ester oder Ether, die verzweigt oder unverzweigt, gesättigt oder ungesättigt sein können, pflanzliche Öle oder Gemische davon.

Ist eine Zubereitung als Aerosol konfektioniert, verwendet man in der Regel die üblichen Treibmittel, wie Alkane, Fluoralkane und Chlorfluoralkane.

Die kosmetische Zubereitung kann auch zum Schutz der Haare gegen photochemische Schäden verwendet werden, um Veränderungen von Farbnuancen, ein Entfärben oder Schäden mechanischer Art zu verhindern. In diesem Fall erfolgt geeignet eine Konfektionierung als Shampoo, Lotion, Gel oder Emulsion zum Ausspülen, wobei die jeweilige Zubereitung vor oder nach dem Shamponieren, vor oder nach dem Färben oder Entfärben bzw. vor oder nach der Dauerwelle aufgetragen wird. Es kann auch eine Zubereitung als Lotion oder Gel zum Frisieren und Behandeln, als Lotion oder Gel zum Bürsten oder Legen einer Wasserwelle, als Haarlack, Dauerwellenmittel, Färbe- oder Entfärbemittel der Haare gewählt werden. Die Zubereitung mit Lichtschutzeigenschaften kann Adjuvantien enthalten, wie Grenzflächen aktive Mittel, Verdickungsmittel, Polymere, weichmachende Mittel, Konservierungsmittel, Schaumstabilisatoren, Elektrolyte, organische Lösungsmittel, Silikonderivate, Öle, Wachse, Antifettmittel, Farbstoffe und/oder Pigmente, die das Mittel selbst oder die Haare färben oder andere für die Haarpflege üblicherweise verwendete Ingredienzien.

Die Glasplättchen können als Füllstoffe beispielsweise in Lippenstiften, Lipgloss, Rouge, Eyeliner, Lidschatten, (Volumen)Mascara, Nagellacke, Tagescremes, Nachtcremes, Körperlotionen, Reinigungsmilch, Körperpuder, Haargele, Haarmasken, Haarspülungen, Haarshampoos, Duschgelen, Duschölen, Badeölen, Sonnenschutz, Prä-Sun- und After-Sun-Präparate, Bräunungslotionen, Bräunungssprays, Make-ups, Lotions, Seifen, Badesalze, Zahnpasta, Gesichtsmasken, Presspuder, lose Puder und Gele, etc., verwendet werden. Die Herstellung derartiger Produkte erfolgt auf eine Weise, wie sie dem Fachmann auf diesem Gebiet bekannt ist.

Gegenstand der Erfindung ist somit die Verwendung der erfindungsgemäßen Glasplättchen als Füllstoff, insbesondere in der pflegenden und dekorativen Kosmetik, sowie in Farben, Lacken, Automobillacken, Pulverlacken, Druckfarben, Sicherheitsdruckfarben, Kunststoffen, Papier, im Papierstrich, in Pigmentanteigungen mit Wasser, organischen und/oder wässrigen Lösemitteln, zur Herstellung von Pigmentpräparationen und Trockenpräparaten, wie z. B. Granulaten. Vorzugsweise werden die Glasplättchen als funktioneller Füllstoff eingesetzt, da die Glasplättchen die Applikations-eigenschaften, das Hautgefühl, den Pay-off und die Verpreßbarkeit von Pudern verbessern können. Des weiteren können sie den fettigen, klebrigen Aspekt der Formulierung reduzieren, verleihen Emulsionen mehr Reichhaltigkeit, können die Viskositätseigenschaften und die Textur beeinflussen und die Trockenzeit von z.B. Mascara, Eyelinern, etc., verbessern.

Die nachfolgenden Beispiele sollen die Erfindung näher erläutern ohne sie jedoch zu beschränken. Vor- und nachstehend sind alle Prozentangaben Gewichtsprozent.

Die Ölzahl ist eine übliche Kennzahl zur Charakterisierung des Ölbedarfs von Pigmenten und wird bestimmt nach DIN EN ISO 787-5: 1995-10.

### Beispiele

Die Zusammensetzungen der Glasplättchen sind der nachfolgenden Tabelle zu entnehmen:

| Zusammensetzung | Beispiel 1 | Beispiel 2 | Beispiel 3 | Beispiel 4 | Beispiel 5 | Beispiel 6 |
|---|---|---|---|---|---|---|
| SiO₂ | 65-70 | 64-70 | 65 | 63.0-67.0 | 65-72 | 52-56 |
| Al₂O₃ | 2-6 | 3-6 | 4 | 3.0-5.0 | 1-7 | 12-16 |
| .CaO | 4-9 | 3-7 | 14 | 4.0-7.0 | 4-11 | 16-25 |
| MgO | 0-5 | 1-4 | 3 | 2.0-4.0 | 0-5 | 0-6 |
| B₂O₃ | 2-7 | 2-5 | 5.5 | 4.0-7.0 | 0-8 | 5-13 |
| Na₂O + K₂O | 9-13 | | 8.5 | | 9-13 | 0-0.8 |
| Na₂O | | 8-13 | | 14.0-17.0 | | |
| K₂O | | 0-3 | | 0-2.0 | | |
| ZnO | 1-6 | 1-5 | 0 | < 0.1 | 0-6 | |
| FeO/Fe₂O₃ | | | 0 | < 0.2 | | |
| TiO₂ | | 0-1 | | | | |
| ZnO | | | | < 0.1 | | |
| BaO | | | | < 0.1 | | |
| F₂ | | | | < 1.0 | | |

### Beispiel 1: Herstellung von 900 nm dicken Glasplättchen aus C-Glas

Ein Glasstrahl aus geschmolzenem C-Glas trifft in senkrechtem Fall auf eine rotierende Tasse. Der Glasstrahl trifft dabei in solcher Art auf die Tasse, dass das geschmolzene Glas am Tassenrand aufwärts transportiert wird und über den Tassenrand tritt. Durch Zentrifugalkräfte wird das Glas horizontal getragen und tritt in einen Spalt zwischen zwei parallel angeordnete Platten ein. Die parallelen Platten befinden sich in einer Vakuumkammer, so dass die entstandene Glaslamelle durch das Vakuum horizontal gehalten wird und so die Platten nicht berührt. Beim Erstarren des Glases bilden sich die planen C-Glasplättchen. Die Plättchen werden in einer herkömmlichen Luftstrahlmühle zu einer Fraktion von 15 - 150 µm vermahlen. Die so hergestellten Glasplättchen haben eine Ölzahl von 75.

### Beispiel 2: Herstellung von 700 nm dicken Glasplättchen aus ECR-Glas

Ein Glasstrahl aus geschmolzenem ECR-Glas trifft in senkrechtem Fall auf eine rotierende Tasse. Der Glasstrahl trifft dabei in solcher Art auf die Tasse, dass das geschmolzene Glas am Tassenrand aufwärts transportiert wird und über den Tassenrand tritt. Durch Zentrifugalkräfte wird das Glas horizontal getragen und tritt in einen Spalt zwischen zwei parallel angeordnete Platten ein. Die parallelen Platten befinden sich in einer Vakuumkammer, so dass die entstandene Glaslamelle durch das Vakuum horizontal gehalten wird und so die Platten nicht berührt. Beim Erstarren des Glases bilden sich die planen ECR-Glasplättchen. Die Plättchen werden in einer herkömmlichen Luftstrahlmühle zu einer Fraktion von 5 - 50 µm vermahlen. Die so hergestellten Glasplättchen haben eine Ölzahl von 80.

### Beispiel 3: Herstellung von 800 nm dicken Glasplättchen aus C-Glas

Ein Glasstrahl aus geschmolzenem C-Glas trifft in senkrechtem Fall auf eine rotierende Tasse. Der Glasstrahl trifft dabei in solcher Art auf die Tasse, dass das geschmolzene Glas am Tassenrand aufwärts transportiert wird und über den Tassenrand tritt. Durch Zentrifugalkräfte wird das Glas horizontal getragen und tritt in einen Spalt zwischen zwei parallel angeordnete Platten ein. Die parallelen Platten befinden sich in einer Vakuumkammer, so dass die entstandene Glaslamelle durch das Vakuum horizontal gehalten wird und so die Platten nicht berührt. Beim Erstarren des Glases bilden sich die planen C-Glasplättchen. Die Plättchen werden in einer herkömmlichen Luftstrahlmühle zu einer Fraktion von 10 -100 µm vermahlen. Die so hergestellten Glasplättchen haben eine Ölzahl von 75.

### Beispiel 4: Herstellung von 700 nm dicken Glasplättchen aus C-Glas

Ein Glasstrahl aus geschmolzenem C-Glas trifft in senkrechtem Fall auf eine rotierende Tasse. Der Glasstrahl trifft dabei in solcher Art auf die Tasse, dass das geschmolzene Glas am Tassenrand aufwärts transportiert wird und über den Tassenrand tritt. Durch Zentrifugalkräfte wird das Glas horizontal getragen und tritt in einen Spalt zwischen zwei parallel angeordnete Platten ein. Die parallelen Platten befinden sich in einer Vakuumkammer, so dass die entstandene Glaslamelle durch das Vakuum horizontal gehalten wird und so die Platten nicht berührt. Beim Erstarren des Glases bilden sich die planen C-Glasplättchen. Die Plättchen werden in einer herkömmlichen Luftstrahlmühle zu einer Fraktion von 15 - 150 µm vermahlen. Die so hergestellten Glasplättchen haben eine Ölzahl von 85.

### Beispiel 5: Herstellung von 750 nm dicken Glasplättchen aus C-Glas

Ein Glasstrahl aus geschmolzenem C-Glas trifft in senkrechtem Fall auf eine rotierende Tasse. Der Glasstrahl trifft dabei in solcher Art auf die Tasse, dass das geschmolzene Glas am Tassenrand aufwärts transportiert wird und über den Tassenrand tritt. Durch Zentrifugalkräfte wird das Glas horizontal getragen und tritt in einen Spalt zwischen zwei parallel angeordnete Platten ein. Die parallelen Platten befinden sich in einer Vakuumkammer, so dass die entstandene Glaslamelle durch das Vakuum horizontal gehalten wird und so die Platten nicht berührt. Beim Erstarren des Glases bilden sich die planen C-Glasplättchen. Die Plättchen werden in einer herkömmlichen Luftstrahlmühle zu einer Fraktion von 10 - 100 µm vermahlen. Die so hergestellten Glasplättchen haben eine Ölzahl von 65.

### Beispiel 6: Herstellung von 800 nm dicken Glasplättchen aus E-Glas

Ein Glasstrahl aus geschmolzenem E-Glas trifft in senkrechtem Fall auf eine rotierende Tasse. Der Glasstrahl trifft dabei in solcher Art auf die Tasse, dass das geschmolzene Glas am Tassenrand aufwärts transportiert wird und über den Tassenrand tritt. Durch Zentrifugalkräfte wird das Glas horizontal getragen und tritt in einen Spalt zwischen zwei parallel angeordnete Platten ein. Die parallelen Platten befinden sich in einer Vakuumkammer, so dass die entstandene Glaslamelle durch das Vakuum horizontal gehalten wird und so die Platten nicht berührt. Beim Erstarren des Glases bilden sich die planen E-Glasplättchen. Die Plättchen werden in einer herkömmlichen Luftstrahlmühle zu einer Fraktion von 5 - 50 µm vermahlen. Die so hergestellten Glasplättchen haben eine Ölzahl von 55.

### Anwendungsbeispiele

### Beispiel A1: Cream Conditioner

| Phase | Rohstoff | INCI | % |
|---|---|---|---|
| A | Wasser | AQUA (WATER) | 75.20 |
| | Glasplättchen gemäß Beispiel 2 (1) | | 5.00 |
| | Luviquat Hold | POLYQUATERNIUM-46 | 5.00 |
| | Luviquat PQ 11 | POL YQUATERNIUM-11 | 2.00 |
| | Butylene Glycol | BUTYLENE GLYCOL | 3.00 |
| B | Cremophor A 6 | CETEARETH-6 AND STEARYL ALCOHOL | 3.00 |
| | Ammonyx 4 | STEARALKONIUM CHLORIDE | 3.00 |
| | Lanette Wax O | CETEARYL ALCOHOL | 2.00 |
| | Eusolex 2292 | OCTYL METHOXYCINNAMATE | 0.10 |
| C | Vitamin E Acetate | TOCOPHERYL ACETATE | 0.50 |
| | Bisabolol | BISABOLOL | 0.10 |
| | Parfum | PARFUM | 0.10 |
| | Germaben II | PROPYLENE GLYCOL, DIAZOLIDINYL UREA, METHYLPARABEN, PROPYLPARABEN | 1.00 |

### Herstellung:

Die Pigmente in Wasser der Phase A dispergieren und die restlichen Rohstoffe zugeben. Nach jeder Zugabe durchrühren und anschließend auf 75 °C erhitzen. Die Rohstoffe der Phase B mischen, auf 75 - 80 °C erhitzen und zu Phase A dazugeben. Mischen bis eine homogene Verteilung vorliegt. Phase C bei 45 °C zugeben.

### Beispiel A2: Duschgel

| Phase | Rohstoff | INCI | % |
|---|---|---|---|
| A | Ronastar® Golden Sparks (1) | CALCIUM ALUMINUM BOROSILICATE, SILICA, CI 77891 TITANIUM DIOXIDE), TIN OXIDE | 0,05 |
| | Glasplättchen gemäß Beispiel 1 (1) | | 0,20 |
| | Keltrol CG-SFT (2) | XANTHAN GUM | 1,10 |
| | Wasser, demineralisiert | WATER, AQUA (WATER) | 54,80 |
| B | Plantacare 2000UP (3) | DECYL GLUCOSIDE | 20,00 |
| | Texapon ASV 50 (3) | SODIUM LAURETH SULFATE, SODIUM LAURETH-8 SODIUM LAURETH SULFATE, SODIUM LAURETH-8 MAGNESIUM LAURETH-8 SULFATE, SODIUM OLETH SULFATE, MAGNESIUM OLETH SULFATE | 3,60 |
| | Bronidox L (3) | PROPYLENE GLYCOL, 5-BROMO-5-NITRO-1,3-DIOXANE | 0,30 |
| | Frag 280851 Fruit Cocktail (4) | PARFUM | 0,20 |
| | 0,1 % Sicovit Chinolingelb 70 E 104 in Wasser (5) | AQUA (WATER), WATER, CI 47005 (ACID YELLO ACID YELLOW 3W 3), | 8,30 |
| | 0,1 % Dragocolor Edelblau in Wasser (6) | AQUA (WATER), WATER, CI 42090 (FD&C BLUE NO. 1), FD&C BLUE NO. 1 | 1,30 |
| C | Citronensäure, Monohydrat (1) | CITRIC ACID | 0,15 |
| | Wasser, demineralisiert | WATER, AQUA (WATER) | 10,00 |

### Herstellung:

Phase A: Wasser im Kessel vorlegen und das Pigment einrühren. Keltrol CG-SFT unter Rühren langsam einstreuen und rühren bis es vollständig gelöst ist (nicht homogenisieren). Die Bestandteile der Phase B einzeln zur Phase A zufügen. Citronensäure Monohydrat in Wasser lösen und zum Ansatz geben und langsam rühren bis alles homogen verteilt ist. Den pH-Wert unter Zugabe von Citronensäure (nach Bedarf) auf 6,0 - 6,5 einstellen.

### Bezugsquellen:

(1) Merck KGaA/Rona®
(2) C. P. Kelco
(3) Cognis GmbH
(4) Drom
(5) BASF AG
(6) Symrise

### Beispiel A3: Lidschatten

| Phase | Rohstoff | INCI | % |
|---|---|---|---|
| A | Xirona® Magic Mauve (1) | SILICA, CI 77891 (TITANIUM DIOXIDE), TIN OXIDE | 27,00 |
| | Microna® Matte Blue (1) | CI 77510 (FERRIC FERROCYANIDE), MICA | 3,00 |
| | Talkum (1) | TALC | 34,50 |
| | Glasplättchen gemäß Beispiel 2 (1) | | 15,00 |
| | Kartoffelstärke (2) | POTATO STARCH, SOLANUM TUBEROSUM (POTATO STARCH) | 7,50 |
| | Magnesiumstearat (1) | MAGNESIUM STEARATE | 2,50 |
| B | Isopropylstearat(3) | ISOPROPYL STEARATE | 9,14 |
| | Cetylpalmitat (1) | CETYL PALMITATE | 0,53 |
| | Ewalin 1751 (4) | PETROLATUM | 0,53 |
| | Parfümöl Elegance + 79228 D MF (5) | PARFUM | 0,20 |
| | Propyl-4-hydroxybenzoat (1) | PROPYLPARABEN | 0,10 |

### Herstellung:

Bestandteile der Phase A zusammen geben und vormischen. Anschließend die Pudermischung unter Rühren tropfenweise mit der geschmolzenen Phase B versetzen. Die Puder werden in Puderpfannen mit großem Durchmesser gefüllt und bei 80 bar verpresst.

### Bezugsquellen:

(1) Merck KGaA/Rona®
(2) Suedstaerke GmbH
(3) Cognis GmbH
(4) H. Erhard Wagner GmbH
(5) Symrise

### Beispiel A4: Faltenreduzierende Tagescreme

| Phase | Rohstoff | INCI | % |
|---|---|---|---|
| A | Ronasphere® LDP (1) | SILICA, CI 77891 (TITANIUM DIOXIDE), CI 77491 (IRON OXIDES) | 5,00 |
| | Veegum HV (2) | MAGNESIUM ALUMINUM SILICATE | 1,00 |
| | Glasplättchen gemäß Beispiel 2 (1) | | 5,00 |
| | Karion F flüssig (1) | SORBITOL | 3,00 |
| | Methyl-4-hydroxybenzoat (1) | METHYLPARABEN | 0,18 |
| | Wasser, demineralisiert | AQUA (WATER) | 51,44 |
| B | Arlacel 165 VP (3) | GLYCERYL STEARATE, PEG-100 STEARATE | 5,00 |
| | Lanette O (4) | CETEARYL ALCOHOL | 1,50 |
| | Miglyol 812 N (5) | CAPRYLIC/CAPRIC TRIGLYCERIDE | 7,00 |
| | Sheabutter fest (6) | BUTYROSPERMUM PARKII (SHEA BUTTER) | 2,00 |
| | Cetiol SN (4) | CETEARYL ISONONANOATE | 7,00 |
| | Eutanol G (4) | OCTYLDODECANOL | 7,50 |
| | Emulgade PL 68/50 (4) | CETEARYL ALCOHOL, CETEARYL GLUCOSIDE | 2,00 |
| | Propyl-4-hydroxybenzoat (1) | PROPYLPARABEN | 0,08 |
| C | Parfümöl 200 530 (7) | PARFUM | 0,20 |
| | Dow Corning 345 (8) | CYCLOMETHICONE | 2,00 |
| | Euxyl K 400 (9 | PHENOXYETHANOL, METHYLDIBROMO GLUTARONITRILE | 0,10 |
| | Citronensäure Monohydrat (1) | CITRIC ACID | 0,00 |

### Herstellung:

Phase B erwärmen bis die Lösung klar ist. Veegum im Wasser der Phase A dispergieren, restliche Rohstoffe zugeben, auf 80 °C erhitzen und Phase B zugeben. Phase A/B homogenisieren. Unter Rühren auf 40 °C abkühlen und Phase C zugeben. Auf Raumtemperatur abkühlen und pH 6,0 einstellen.

### Bezugsquellen:

(1) Merck KGaA/Rona®
(2) Vanderbilt
(3) Uniqema
(4) Cognis GmbH
(5) Sasol Germany GmbH
(6) H. Erhard Wagner GmbH
(7) Fragrance Resources
(8) Dow Corning
(9) Schülke & Mayr GmbH

### Beispiel A5: Sparkling Body Cream

| Phase | Rohstoff | INCI | % |
|---|---|---|---|
| A | Ronastar® Copper Sparks (1) | CALCIUM ALUMINUM BOROSILICATE, SILICA, CI 77891 (TITANIUM DIOXIDE), TIN OXIDE | 3,00 |
| | Glasplättchen gemäß Beispiel 5(1) | | 3,00 |
| | Wasser, demineralisiert | WATER, AQUA (WATER) | 36,60 |
| | Carbopol Ultrez 21 (2) | ACRYLATES/C10-30 ALKYL ACRYLATE CROSSPOLYMER | 0,60 |
| | Citronensäure Monohydrat (1) | CROSSPOLYMER | 0,00 |
| B | Wasser, demineralisiert | WATER, AQUA (WATER) | 26,35 |
| | 1,2-Propandiol (1) | PROPYLENE GLYCOL | 3,00 |
| | RonaCare® Allantoin (1) | PROPYLENE GLYCOL | 0,20 |
| C | Paraffin flüssig (1) | PARAFFINUM LIQUIDUM (MINERAL OIL), MINERAL OIL | 10,00 |
| | Cetiol V (3) | DECYL OLEATE | 6,00 |
| | Hostaphat KL 340 D (4) | TRILAURETH-4 PHOSPHATE | 3,00 |
| | Cetylalkohol (1) | CETYLALCOHOL | 2,00 |
| | Phenonip (5) | PHENOXYETHANOL, BUTYLPARABEN, ETHYLPARABEN, PROPYLPARABEN, METHYLPARABEN | 0,50 |
| D | Wasser, demineralisiert | WATER, AQUA (WATER) | 3,50 |
| | Triethanolamin | TRIETHANOLAMINE | 0,35 |
| E | Germall 115 (6) | IMIDAZOLIDINYL UREA | 0,30 |
| | Parfümöl Vogue (7 | PARFUM | 0,10 |
| | Wasser, demineralisiert | WATER, AQUA (WATER) | 1,50 |

### Herstellung:

Das Perlglanzpigment im Wasser der Phase A dispergieren. Eventuell mit einigen Tropfen Citronensäure ansäuern, um die Viskosität zu vermindern. Carbopol unter Rühren einstreuen. Nach vollständiger Lösung die vorgelöste Phase B langsam einrühren. Phase A/B und Phase C auf 80 °C erhitzen, Phase C in Phase A/B einrühren, homogenisieren mit Phase D neutralisieren, nochmals homogenisieren und unter Rühren abkühlen. Bei 40 °C im Wasser der Phase E Germall 115 lösen, unter Rühren hinzugeben. Danach Parfümöl zugeben und unter Rühren auf Raumtemperatur abkühlen.

### Bezugsquellen:

(1) Merck KGaA/Rona®
(2) Noveon
(3) Cognis GmbH
(4) Clariant GmbH
(5) Nipa Laboratorien GmbH
(6) ISP Global Technologies
(7) Drom

### Beispiel A6: Creamy Eye Shadow

| Phase | Rohstoff | INCI | % |
|---|---|---|---|
| A | Xirona® Golden Sky (1) | SILICA, CI 77891 (TITANIUM DIOXIDE), TIN OXIDE | 14,00 |
| | Glasplättchen gemäß Beispiel 3 (1) | | 6,00 |
| | Unipure Green LC 789 CF (2) | CI 77289 (CHROMIUM HYDROXIDE GREEN) | 3,00 |
| B | Crodamol PMP (3) | PPG-2 MYRISTYL ETHER PROPIONATE | 41,58 |
| | Syncrowax HGLC (3) | C18-36 ACID TRIGLYCERIDE | 11,00 |
| | Syncrowax HRC (3) | TRIBEHENIN | 3,30 |
| | Miglyol 812 N (4) | CAPRYLIC/CAPRIC TRIGLYCERIDE | 15,40 |
| | Stearinsäure (1) | STEARIC ACID | 3,30 |
| | Antaron V-216 (5) | PVP/HEXADECENE COPOLYMER | 2,20 |
| | Oxynex® K flüssig (1) | PVP/HEXADECENE COPOLYMER, ASCORBIC ACID, CITRIC ACID | 0,11 |
| | Propyl-4-hydroxybenzoat (1) | PROPYLPARABEN | 0,11 |

### Herstellung:

Phase B auf ca. 80 °C erhitzen bis alles geschmolzen ist und auf 65 °C abkühlen. Unter Rühren werden nun das Perlglanzpigment und das vermahlene Chromoxid der Phase A zugegeben. Der Lidschatten wird bei 65 °C abgefüllt.

### Bezugsquellen:

(1) Merck KGaA/Rona®
(2) Les Colorants Wackherr
(3) Croda GmbH
(4) Sasol Germany GmbH
(5) ISP Global Technologies

### Beispiel A7: Hair Styling Gel

| Phase | Rohstoff | INCI | % |
|---|---|---|---|
| A | Ronastar® Blue Sparks (1) | CALCIUM ALUMINUM BOROSILICATE, CI 77891 CALCIUM ALUMINUM BOROSILICATE, CI 77891 | 2,55 |
| | Xirona® Silver (1) | ALUMINA, CI 77891 (TITANIUM DIOXIDE), TIN OXIDE | 0,40 |
| | Colorona® Patina Silver (1) | MICA, CI 77499 (IRON OXIDES), CI 77891 (TITANIUM DIOXIDE | 0,05 |
| | Glasplättchen gemäß | | 3,00 |
| | Beispiel 2 (1) | | |
| | Carbopol Ultrez 21 (2) | ACRYLATES/C10-30 ALKYL ACRYLATE CROSSPOLYMER | 0,90 |
| | Wasser, demineralisiert | WATER, AQUA (WATER) | 47,00 |
| B | Luviskol K 30 Pulver (3) | PVP | 2,00 |
| | Germaben II (4) | PROPYLENE GLYCOL, DIAZOLIDINYL UREA, METHYLPARABEN, PROPYLPARABEN | 1,00 |
| | Triethanolamin reinst (1) | TRIETHANOLAMINE | 2,16 |
| | Wasser, demineralisiert | WATER, AQUA (WATER) | 40,94 |

### Herstellung:

Die Perlglanzpigmente im Wasser der Phase A dispergieren und das Carbopol unter Rühren einstreuen. Nach vollständiger Lösung die vorgelöste Phase B langsam einrühren.

### Bezugsquellen:

(1) Merck KGaA/Rona®
(2) Noveon
(3) BASF AG
(4) ISP Global Technologies

### Beispiel A8: Shampoo

| Phase | Rohstoff | INCI | % |
|---|---|---|---|
| A | Glasplättchen gemäß Beispiel 4 (1) | | 3,00 |
| | Carbopol ETD 2020 (2) | ACRYLATES/C10-30 ALKYL ACRYLATE CROSSPOLYMER | 0,90 |
| | Wasser, demineralisiert | AQUA (WATER) | 60,60 |
| B | Triethanolamin reinst (1) | TRIETHANOLAMINE | 0,90 |
| | Wasser, demineralisiert | AQUA (WATER) | 10,00 |
| C | Plantacare 2000 UP (3) | DECYL GLUCOSIDE | 20,00 |
| | Texapon ASV 50 (3) | SODIUM LAURETH SULFATE, SODIUM LAURETH-8, SULFATE, MAGNESIUM LAURETH SULFATE, SULFATE, MAGNESIUM LAURETH SULFATE, SULFATE, MAGNESIUM OLETH SULFATE | 4,35 |
| | Bronidox L (3) | PROPYLENE GLYCOL, 5-BROMO-5-NITRO-1,3-DIOXANE | 0,20 |
| | Parfümöl 200 524 (4) | PARFUM | 0,05 |
| | Farbstofflösung (q.s.) | | 0,00 |

### Herstellung:

Für Phase A den Füllstoff in das Wasser einrühren. Mit einigen Tropfen Citronensäure (10%ig) ansäuern um die Viskosität zu vermindern und das Carbopol unter Rühren langsam einstreuen. Nach vollständiger Lösung langsam Phase B zugeben. Nacheinander werden nun die Bestandteile der Phase C zugegeben. Den pH-Wert auf 6,0 - 6,5 einstellen.

### Bezugsquellen:

(1) Merck KGaA/Rona®
(2) Noveon
(3) Cognis GmbH
(4) Fragrance Resources

### Beipiel A9: Shimmering Body Powder

| Phase | Rohstoff | INCI | % |
|---|---|---|---|
| A | Timiron® Karat Gold MP-24 (1) | MICA, CI 77891 (TITANIUM DIOXIDE), CI 77491 (IRON OXIDES) | 10,00 |
| B | Microna® Matte Red (1) | CI 77491 (IRON OXIDES), MICA | 1,00 |
| | Microna® Matte Yellow (1) | MICA, CI 77492 (IRON OXIDES) | 1,00 |
| | Ronasphere® LDP (1) | SILICA, CI 77891 (TITANIUM DIOXIDE), CI 77491 (IRON OXIDES | 4,00 |
| | Talkum (1) | TALC | 25,00 |
| | Glasplättchen gemäß Beispiel 6 (1) | | 15,00 |
| | Weißer Ton (1) | KAOLIN | 14,70 |
| | Mica M (1) | MICA | 15,00 |
| | Silk Mica (1) | MICA | 9,50 |
| | Propyl-4-hydroxybenzoat (1) | PROPYLPARABEN | 0,30 |
| C | Cetiol SQ (2) | SQUALANE | 2,00 |
| | Miglyol 812 N (3) | CAPRYLIC/CAPRIC TRIGLYCERIDE | 2,00 |
| | RonaCare® Tocopherolacetat (1) | TOCOPHERYL ACETATE | 0,20 |
| | Fragrance Baby Cotton DC10122/1 (4) | PARFUM | 0,30 |

### Herstellung:

Alle Bestandteile der Phase B zusammen einwiegen und einem Mixer homogen vermahlen. Anschließend Phase C zugeben und weiter mixen, dann Phase A zufügen und kurz vermahlen, bis das Perlglanzpigment gleichmäßig verteilt ist.

### Bezugsquellen:

(1) Merck KGaA/Rona®
(2) Cognis GmbH
(3) Sasol Germany GmbH
(4) Symrise

### Beispiel A10: Long Lasting Lipgloss

| Phase | Rohstoff | INCI | % |
|---|---|---|---|
| A | Xirona® Le Rouge (1) | SILICA, CI 77491 (IRON OXIDES), | 7,70 |
| | Glasplättchen gemäß Beispiel 2(1) | | 3,00 |
| | Ronastar® Red Sparks (1) | CALCIUM ALUMINUM BOROSILICATE, CI 77891 CALCIUM ALUMINUM BOROSILICATE, CI 77891 | 3,30 |
| B | Indopol H 100 (2) | POLYBUTENE | 29,00 |
| | Jojoba Glaze LV (3) | SIMMONDSIA CHINENSIS (JOJOBA), JOJOBA, SEED OIL, ETHYLENE/PROPYLENE/ST YRENE COPOLYMER, BUTYLENE/ETHYLENE/STY RENE COPOLYMER | 19,00 |
| | Jojoba Glaze HV (3) | SIMMONDSIA CHINENSIS (JOJOBA), JOJOBA, SEED OIL, ETHYLENE/PROPYLENE/ST YRENE COPOLYMER, BUTYLENE/ETHYLENE/STY RENE COPOLYMER | 10,00 |
| | Rizinusöl (4) | CASTOR OIL, RICINUS COMMUNIS (CASTOR OIL) | 20,15 |
| | Bienenwachs gebleicht (1) | BEESWAX, CERA ALBA (BEESWAX) | 4,00 |
| | Propyl-4-hydroxybenzoat (1) | PROPYLPARABEN | 0,10 |
| | Oxynex® K flüssig (1) | PEG-8, TOCOPHEROL, ASCORBYL PALMITATE, ASCORBIC ACID, CITRIC ACID | 0,05 |
| | Rubis Covapate W 4765 (5) | RICINUS COMMUNIS (CASTOR OIL), CASTOR OIL, CI 15850 (D&C RED NO. 7 CALCIUM LAKE), D&C RED NO. 7 CALCIUM LAKE | 2,00 |
| C | Neosil CT11 (6) | SILICA | 1,50 |
| | Fragrance Tendresse 75418C (7 | PARFUM | 0,20 |

### Herstellung:

Alle Bestandteile der Phase B werden zusammen eingewogen, auf 80 °C erhitzt und gut durchgerührt. Die Pigmente der Phase A unterrühren, das Neosil unter Rühren einstreuen und zum Schluß das Parfüm zugeben. Die homogene Mischung in Container abfüllen.

### Bezugsguellen:

(1) Merck KGaA/Rona®
(2) BP Lavera Sud
(3) Desert Whale
(4) Henry Lamotte GmbH
(5) Les Colorants Wackherr
(6) Ineos Silicas Limited
(7) Symrise

### Beispiel A11: Nagellack

| Phase | Rohstoff | INCI | % |
|---|---|---|---|
| A | Xirona® Le Rouge (1) | SILICA, CI 77491 (IRON OXIDES), | 1,75 |
| | Glasplättchen gemäß Beispiel 2 (1) | | 1,00 |
| | Ronastar® Red Sparks (1) | CALCIUM ALUMINUM BOROSILICATE, CI 77891 CALCIUM ALUMINUM BOROSILICATE, CI 77891 | 0,25 |
| | Coloring Base Ref. 690 (2) | BUTYL ACETATE, ETHYL ACETATE, NITROCELLULOSE, PHTHALIC, ANHYDRIDE/TRIMELLITIC ANHYDRIDE/GL YCOLS COPOLYMER, CI 15850 (D&C RED NO. 7 CALCIUM LAKE), D&C RED NO. 7 CALCIUM LAKE, ISOPROPYL ALCOHOL, ACETYL TRIBUTYL CITRATE, STEARALKONIUM HECTORITE | 2,00 |
| | Thixotrope Nagellack-Base 155 (2) | BUTYL ACETATE, ETHYL ACETATE, NITROCELLULOSE, ACETYL TRIBUTYL CITRATE, PHTHALIC ANHYDRIDE/TRIMELLITIC ANHYDRIDE/GL YCOLS COPOLYMER, ISOPROPYL ALCOHOL, STEARALKONI UM HECTORITE, ADIPIC ACID/FUMARIC ACID/PHTHALIC ACID/TRICYCLODECANE DIMETHANOL COPOLYMER, CITRIC ACID | 95,00 |

### Herstellung:

Die Pigmente werden zusammen mit der Lackbase eingewogen, gut mit einem Spatel von Hand vermischt und anschließend 10 min bei 1000 Upm gerührt.

### Bezugsquellen:

(1) Merck KGaA/Rona®
(2) Durlin/BergeracNC

### Beispiel A12: Volume Mascara

| Phase | Rohstoff | INCI | % |
|---|---|---|---|
| A | Ronastar® Silver (1) | CALCIUM ALUMINUM BOROSILICATE, SILICA, CI 77891 (TITANIUM DIOXIDE), TIN OXIDE | 4,00 |
| | Timiron® Splendid Blue (1) | CI 77891 (TITANIUM DIOXIDE), MICA, SILICA | 3,00 |
| | Mica Black (1) | CI 77499 (IRON OXIDES), MICA, CI 77891 (TITANIUM DIOXIDE) | 4,50 |
| | Glasplättchen gemäß, Beispiel 5 (1) | | 4,00 |
| | Satin Mica (1) | MICA | 2,00 |
| B | Dermacryl 79 (2) | ACRYLATES/OCTYLACRYLAM IDE COPOLYMER | 3,50 |
| | Bienenwachs gebleicht (1) | BEESWAX, CERA ALBA (BEESW AX) | 3,00 |
| | Syncrowax HRC (3) | TRIBEHENIN | 3,50 |
| | Stearinsäure (1) | STEARIC ACID | 5,00 |
| | Tegin M (4) | GLYCERYL STEARATE | 3,50 |
| | Tegosoft CT (4) | CAPRYLIC/CAPRIC TRIGLYCERIDE | 2,50 |
| | Dow Corning 556 (5) | PHENYL TRIMETHICONE | 2,00 |
| | RonaCare® Tocopherolacetat (1) | PHENYL TRIMETHICONE | 0,50 |
| | Phenonip (6) | PHENOXYETHANOL, BUTYLPARABEN, ETHYLPARABEN, PROPYLPARABEN, METHYLPARABEN | 0,80 |
| C | Wasser, demineralisiert | WATER, AQUA (WATER) | 53,65 |
| | AMP Ultra PC 1000 (7) | AMINOMETHYL PROPANOL | 1,25 |
| | 1,3-Butandiol (1) | BUTYLENE GLYCOL | 1,00 |
| | RonaCare® Biotin Plus (1) | UREA, DISODIUM PHOSPHATE, BIOTIN, CITRIC ACID | 0,50 |
| D | Germall 115 (8) | IMIDAZOLIDINYL UREA | 0,30 |
| | Wasser, demineralisiert | WATER, AQUA (WATER) | 1,50 |

### Herstellung:

Alle Bestandteile der Phase B außer Demacryl 79 zusammen bei ca. 85 °C aufschmelzen, unter rühren Demacryl 79 zugeben und 20 min rühren lassen bis alles homogen verteilt ist. Die Bestandteile der Phase C auf ca. 85 °C erhitzen. Die Perlglanzpigmente der Phase A in Phase C einrühren. Phase C zu Phase B geben, weiter rühren und 1 min mit dem Ultra-Turrax T25 bei 8000 upm homogenisieren. Unter rühren abkühlen lassen und bei 40 °C Phase D zufügen.

### Bezugsquellen:

(1) Merck KGaA/Rona®
(2) National Starch & Chemical
(3) Croda GmbH
(4) Degussa-Goldschmidt AG
(5) Dow Corning
(6) Nipa Laboratorien GmbH
(7) Angus Chemie GmbH
(8) ISP Global Technologies

### Beispiel A13: Shimmering Foundation

| Phase | Rohstoff | INCI | % |
|---|---|---|---|
| A | Extender W (1) | MICA, CI 77891 (TITANIUM DIOXIDE) | 9,00 |
| | Microna® Matte Yellow (1) | MICA, CI 77492 (IRON OXIDES) | 4,00 |
| | Microna® Matte Red (1) | CI 77491 (IRON OXIDES), MICA | 0,40 |
| | Microna® Matte Black (1) | CI 77499 (IRON OXIDES), MICA | 0,30 |
| | Timiron® Supersheen MP-1001(1) | MICA, CI 77891 (TITANIUM DIOXIDE) | 4,50 |
| | Glasplättchen gemäß Beispiel 2 (1) | | 8,00 |
| B | Blanose 7 HF (2) | CELLULOSE GUM | 0,20 |
| | Veegum (3) | MAGNESIUM ALUMINUM SILICATE | 1,00 |
| | Texapon K 1296 (4) | SODIUM LAURYL SULFATE | 0,60 |
| | Triethanolamin reinst (1) | TRIETHANOLAMINE | 0,50 |
| | Titriplex® III (1) | DISODIUM EDTA | 0,25 |
| | Methyl-4-hyd roxybenzoat (1) | METHYLPARABEN | 0,15 |
| | 1,2-Propandiol (1) | METHYLPARABEN | 10,90 |
| | Wasser, demineralisiert | AQUA (WATER) | 39,95 |
| C | Isopropylmyristat (4) | ISOPROPYL MYRISTATE | 8,00 |
| | Paraffin dünnflüssig (1) | PARAFFINUM LIQUIDUM (MINERAL OIL) | 3,60 |
| | Crodamol SS (5) | PARAFFINUM LIQUIDUM (MINERAL OIL) | 2,60 |
| | Monomuls 60-35 C (4) | HYDROGENATED PALM GLYCERIDES | 1,70 |
| | Stearinsäure (1) | STEARIC ACID | 1,50 |
| | Eusolex® 6300(1) | 4-METHYLBENZYLIDENE CAMPHOR | 1,30 |
| | Eusolex® 4360 (1) | BENZOPHENONE-3 | 0,50 |
| | RonaCare® Tocopherolacetat (1) | TOCOPHERYL ACETATE | 0,50 |
| | Magnesiumstearat (1) | MAGNESIUM STEARATE | 0,10 |
| | Propyl-4-hydroxybenzoat (1) | PROPYLPARABEN | 0,05 |
| D | Parfümöl 200 529 (6) | PARFUM | 0,20 |
| | Euxyl K 400 (7) | PHENOXYETHANOL, METHYLDIBROMO GLUTARONITRILE | 0,20 |

### Herstellung:

Alle Bestandteile der Phase C bei ca. 75 °C aufschmelzen und rühren, bis alles geschmolzen ist. Das Wasser der Phase B kalt vorlegen, Blanose mit dem Turrax einhomogenisieren, Veegum einstreuen und erneut homogenisieren. Auf 75 °C erwärmen und unter Rühren die übrigen Bestandteile darin lösen. Inhaltsstoffe der Phase A einrühren. Bei 75 °C unter Rühren die Phase C zugeben und 2 min. homogenisieren. Die Masse unter Rühren auf 40 °C abkühlen, Phase D zugeben. Unter Rühren auf Raumtemperatur weiter abkühlen und auf pH 6,0 - 6,5 einstellen (z. B. mit Citronensäurelösung).

### Bezugsquellen:

(1) Merck KGaA/Rona®
(2) Aqualon GmbH
(3) Vanderbilt
(4) Cognis GmbH
(5) Croda GmbH
(6) Fragrance Resources
(7) Schülke & Mayr GmbH

### Beispiel A14: Getönte Tagescreme mit UV-Schutz

| Phase | Rohstoff | INCI | % |
|---|---|---|---|
| A | Eusolex® 2292 (1) | ETHYLHEXYL METHOXYCINNAMATE, BHT | 3,00 |
| | Eusolex® 4360 (1) | BENZOPHENONE-3 | 3,00 |
| | Arlacel 165 VP (2) | GLYCERYL STEARATE, PEG-100 STEARATE | 5,00 |
| | Eusolex® HMS (1) | HOMOSALATE | 5,00 |
| | Arlacel 165 VP (2) | GLYCERYL STEARATE, PEG-100 STEARATE | 3,00 |
| | Montanov 68 (3) | CETEARYL ALCOHOL, CETEARYL GLUCOSIDE | 3,00 |
| | Dow Corning 345 (4) | CYCLOMETHICONE | 0,50 |
| | Eutanol G (5) | OCTYLDODECANOL | 2,00 |
| | Propyl-4-hydroxybenzoat (1) | PROPYLPARABEN | 0,05 |
| B | Eusolex® T-2000 (1) | TITANIUM DIOXIDE, ALUMINA, SIMETHICONE | 3,00 |
| | Extender W (1) | MICA, CI 77891 (TITANIUM DIOXIDE) | 4,00 |
| | Microna® Matte Yellow (1) | MICA, CI 77492 (IRON OXIDES) | 2,00 |
| | Microna® Matte Orange (1) | MICA, CI 77491 (IRON OXIDES) | 0,20 |
| | Microna® Matte Red (1) | CI 77491 (IRON OXIDES), MICA | 0,20 |
| | Microna® Matte Black (1) | CI 77499 (IRON OXIDES), MICA | 0,20 |
| | Glasplättchen gemäß Beispiel 2 (1) | | 3,00 |
| | Karion FP, flüssig (1) | SORBITOL | 5,00 |
| | RonaCare® Allantoin (1) | ALLANTOIN | 0,50 |
| | Keltrol T (6) | XANTHAN GUM | 0,20 |
| | Chemag 2000 (gestrichen) (7) | XANTHAN GUM | 0,30 |
| | Euxyl K 400 (8) | PHENOXYETHANOL, METHYLDIBROMO GLUTARONITRILE | 0,10 |
| | Methyl-4-hydroxybenzoat (1) | METHYLPARABEN | 0,15 |
| | Wasser, demineralisiert | AQUA (WATER) | 56,60 |

### Herstellung:

Alle Bestandteile außer Keltrol T im Wasser von Phase B dispergieren. Das Keltrol unter Rühren in Phase B einstreuen und nach 15 Minuten auf 80 °C erhitzen. Phase A auf 75 °C erhitzen. Langsam Phase B in Phase A einrühren und homogenisieren. Unter Rühren abkühlen.

### Bezugsguellen:

(1) Merck KGaA/Rona®
(2) Uniqema
(3) Seppic
(4) Dow Corning
(5) Cognis GmbH
(6) C. P. Kelco
(7) Chemag AG
(8) Schülke & Mayr GmbH

### Beispiel A15: Pflegende Selbstbräunungscreme (O/W)

| Phase | Rohstoff | INCI | [%] |
|---|---|---|---|
| A | | | |
| | Montanov 68 (1) | CETEARYL ALCOHOL, CETEARYL GLUCOSIDE | 4,00 |
| | Span 60 (2) | SORBITAN STEARATE | 1,50 |
| | Lanette O (3) | CETEARYL ALCOHOL | 1,00 |
| | Cosmacol ELI (4) | C12-13 ALKYL LACTATE | 3,00 |
| | Cosmacol EMI (4) | DI-C12-13 ALKYL MALATE | 1,50 |
| | Arlamol HD (2) | ISOHEXADECANE | 3,00 |
| | Dow Corning 9040 Silicone Elastomer Blend (5) | CYCLOMETHICONE, DIMETHICONE CROSSPOLYMER | 1,00 |
| | RonaCare® Tocopherol Acetate (6) | TOCOPHERYL ACETATE | 0,50 |
| | Propyl-4-hydroxybenzoate (6) | PROPYLPARABEN | 0,05 |
| B | | | |
| | RonaCare® Ectoin (6) | ECTOIN | 0,50 |
| | Glasplättchen gemäß Beispiel 2 (1) | | 2,00 |
| | Ronastar® Silver (1) | CALCIUM ALUMINUM BOROSILICATE, SILICA, CI 77891 (TITANIUM DIOXIDE), TIN OXIDE | 2,00 |
| | Glycerol, anhydrous (6) | GLYCERIN | 2,00 |
| | FD&C Yellow No. 6 W082 (8) | Cl 15985 | 0,01 |
| | Methyl-4-hydroxybenzoate (6) | METHYLPARABEN | 0,15 |
| | Water, demineralized | AQUA (WATER) | 62,09 |
| C | | | |
| | Sepigel 305 (1) | LAURETH-7, POLYACRYLAMIDE, C13-14 ISOPARAFFIN | 0,50 |
| D | | | |
| | Dihydroxyacetone (6) | DIHYDROXYACETONE | 5,00 |
| | Water, demineralized | AQUA (WATER) | 10,00 |
| E | | | |
| | Fragrance Babylon (9) | Parfum | 0,20 |

### Herstellung:

Phase A und B werden separat auf 75 °C erwärmt. Dann wird zu Phase A langsam Phase B unter Rühren zugemischt. Bei 60 °C wird Phase C zu der Phase A/B mit einem Handrührer zugemischt und homogenisiert. Auf 40 °C abkühlen lassen und die Phase D und Phase E unterrühren.

### Bezugsquellen:

(1) Seppic
(2) Uniqema
(3) Cognis GmbH
(4) Condea Chimica D.A.C. S.p.A.
(5) Dow Corning
(6) Merck KGaA/Rona®
(7) D.D.Williamson
(8) Les Colorants Wackherr SA
(9) Drom

Die kosmetischen Formulierungen der Beispiele A1 bis A15 zeichnen sich durch eine sehr gute Hautverträglichkeit, ein gutes Hautgefühl und die guten Applikationseigenschaften aus.

## Patentansprüche

1. Verwendung von Glasplättchen mit einer Dicke von < 1 µm und einer Teilchengröße von 1-150 µm, einer Brechzahl von 1,2-2,1 und einer Transparenz von ≥ 90 %, in
- Emulsionen 0,1 - 30 Gew.%,
- pigmenthaltigen Emulsionen 0,1 - 50 Gew.%,
- Zahnpasta 0,1 - 60 Gew.%,
- wasserfreien auf Öl-/Wachs-basierenden Produkten 0,1 - 75 Gew.%, oder
- Puderprodukten 0,1 - 95 Gew.%,
bezogen auf die Gesamtformulierung, wobei die Zusammensetzungen der Glasplättchen der nachfolgenden Tabelle zu entnehmen sind:
| Zusammensetzung | Beispiel 1 | Beispiel 2 | Beispiel 3 | Beispiel 4 | Beispiel 5 |
|---|---|---|---|---|---|
| SiO₂ | 65-70 | 64-70 | 65 | 63.0-67.0 | 65-72 |
| Al₂O₃ | 2-6 | 3-6 | 4 | 3.0-5.0 | 1-7 |
| CaO | 4-9 | 3-7 | 14 | 4.0-7.0 | 4-11 |
| MgO | 0-5 | 1-4 | 3 | 2.0-4.0 | 0-5 |
| B₂O₃ | 2-7 | 2-5 | 5.5 | 4.0-7.0 | 0-8 |
| Na₂O + K₂O | 9-13 | | 8.5 | | 9-13 |
| Na₂O | | 8-13 | | 14.0-17.0 | |
| K₂O | | 0-3 | | 0-2.0 | |
| ZnO | 1-6 | 1-5 | 0 | < 0.1 | 0-6 |
| FeO/Fe₂O₃ | | | 0 | < 0.2 | |
| TiO₂ | | 0-1 | | | |
| ZnO | | | | < 0.1 | |
| BaO | | | | < 0.1 | |
| F₂ | | | | < 1.0 | |

2. Verwendung nach Anspruch 1, **dadurch gekennzeichnet, dass** die Glasplättchen mit anorganischen Farbmitteln masseeingefärbt sind.

3. Verwendung nach Anspruch 1 oder 2, **dadurch gekennzeichnet, dass** es sich bei den Farbmitteln um die Kationen oder komplexe Anionen der Elemente Cu, Cr, Mn, Fe und Co und/oder deren Kombinationen oder TiO₂ oder elementare Edelmetalle handelt.

4. Verwendung nach einem oder mehreren der Ansprüche 1 bis 3, **dadurch gekennzeichnet, dass** die Glasplättchen ein Aspect Ratio von 1 bis 1500 aufweisen.

5. Verwendung nach einem oder mehreren der Ansprüche 1 bis 4, **dadurch gekennzeichnet, dass** die Glasplättchen eine Ölzahl (bestimmt nach DIN ISO 787-5:1995-10) von 20 bis 130 aufweisen.

6. Verwendung nach einem oder mehreren der Ansprüche 1 bis 5, **dadurch gekennzeichnet, dass** die Glasplättchen amorph sind.

7. Verwendung nach einem oder mehreren der Ansprüche 1 bis 6, **dadurch gekennzeichnet, dass** die Glasplättchen in den Formulierungen mit Wasser, Alkohol, Polyolen, polaren und unpolaren Ölen, Fetten, Wachsen, Filmbildnern, Polymeren, CoPolymeren, Tensiden, Radikalfängern, Antioxidantien, Stabilisatoren, Geruchsverstärkern, Silikonölen, Emulgatoren, Duftstoffen, Lösemitteln, Konservierungsmitteln, Verdickern, rheologischen Zusatzstoffen und/oder oberflächenaktiven Hilfsmitteln kombiniert werden.

8. Verwendung nach einem oder mehreren der Ansprüche 1 bis 7, **dadurch gekennzeichnet, dass** die Glasplättchen eine Dicke von 100 nm - 1 µm aufweisen.

9. Verwendung nach einem oder mehreren der Ansprüche 1 bis 8, **dadurch gekennzeichnet, dass** die Glasplättchen eine Dicke von 600 nm - 1 µm aufweisen.

## Claims

1. Use of glass flakes having a thickness of < 1 µm and a particle size of 1-150 µm, a refractive index of 1.2-2.1 and a transparency of ≥ 90% in
- emulsions 0.1 - 30% by weight,
- pigment-containing emulsions 0.1 - 50% by weight,
- toothpastes 0.1 - 60% by weight,
- water-free oil/wax-based products 0.1 - 75% by weight, or
- powder products 0.1 - 95% by weight,
based on the entire formulation, where the compositions of the glass flakes are shown in the following table:
| Composition | Example 1 | Example 2 | Example 3 | Example 4 | Example 5 |
|---|---|---|---|---|---|
| SiO₂ | 65-70 | 64-70 | 65 | 63.0-67.0 | 65-72 |
| Al₂O₃ | 2-6 | 3-6 | 4 | 3.0-5.0 | 1-7 |
| CaO | 4-9 | 3-7 | 14 | 4.0-7.0 | 4-11 |
| MgO | 0-5 | 1-4 | 3 | 2.0-4.0 | 0-5 |
| B₂O₃ | 2-7 | 2-5 | 5.5 | 4.0-7.0 | 0-8 |
| Na₂O + K₂O | 9-13 | | 8.5 | | 9-13 |
| Na₂O | | 8-13 | | 14.0-17.0 | |
| K₂O | | 0-3 | | 0-2.0 | |
| ZnO | 1-6 | 1-5 | 0 | < 0.1 | 0-6 |
| FeO/Fe₂O₃ | | | 0 | < 0.2 | |
| TiO₂ | | 0-1 | | | |
| ZnO | | | | < 0.1 | |
| BaO | | | | < 0.1 | |
| F₂ | | | | < 1.0 | |

2. Use according to Claim 1, **characterised in that** the glass flakes have been mass-coloured with inorganic colorants.

3. Use according to Claim 1 or 2, **characterised in that** the colorants are the cations or complex anions of the elements Cu, Cr, Mn, Fe and Co and/or combinations thereof or TiO₂ or elemental noble metals.

4. Use according to one or more of Claims 1 to 3, **characterised in that** the glass flakes have an aspect ratio of 1 to 1500.

5. Use according to one or more of Claims 1 to 4, **characterised in that** the glass flakes have an oil absorption value (determined in accordance with DIN ISO 787-5:1995-10) of 20 to 130.

6. Use according to one or more of Claims 1 to 5, **characterised in that** the glass flakes are amorphous.

7. Use according to one or more of Claims 1 to 6, **characterised in that** the glass flakes are combined in the formulations with water, alcohol, polyols, polar and nonpolar oils, fats, waxes, film formers, polymers, copolymers, surfactants, free-radical scavengers, antioxidants, stabilisers, odour enhancers, silicone oils, emulsifiers, fragrances, solvents, preservatives, thickeners, rheological additives and/or surface-active assistants.

8. Use according to one or more of Claims 1 to 7, **characterised in that** the glass flakes have a thickness of 100 nm - 1 µm.

9. Use according to one or more of Claims 1 to 8, **characterised in that** the glass flakes have a thickness of 600 nm - 1 µm.

## Revendications

1. Utilisation de paillettes de verre ayant une épaisseur de < 1 µm et une taille de particules de 1-150 µm, un indice de réfraction de 1,2-2,1 et une transparence de ≥ 90% dans
- des émulsions, 0,1 - 30% en poids,
- des émulsions contenant des pigments, 0,1 - 50% en poids,
- des dentifrices, 0,1 - 60% en poids,
- des produits à base de cire/d'huile exempts d'eau, 0,1 - 75% en poids, ou
- des produits pulvérulents, 0,1 - 95% en poids,
sur la base de l'ensemble de la formulation, où les compositions des paillettes de verre sont illustrées dans le tableau suivant :
| Composition | Exemple 1 | Exemple 2 | Exemple 3 | Exemple 4 | Exemple 5 |
|---|---|---|---|---|---|
| SiO₂ | 65-70 | 64-70 | 65 | 63,0-67,0 | 65-72 |
| Al₂O₃ | 2-6 | 3-6 | 4 | 3,0-5,0 | 1-7 |
| CaO | 4-9 | 3-7 | 14 | 4,0-7,0 | 4-11 |
| MgO | 0-5 | 1-4 | 3 | 2,0-4,0 | 0-5 |
| B₂O₃ | 2-7 | 2-5 | 5,5 | 4,0-7,0 | 0-8 |
| Na₂O + K₂O | 9-13 | | 8,5 | | 9-13 |
| Na₂O | | 8-13 | | 14,0-17,0 | |
| K₂O | | 0-3 | | 0-2,0 | |
| ZnO | 1-6 | 1-5 | 0 | < 0,1 | 0-6 |
| FeO/Fe₂O₃ | | | 0 | < 0,2 | |
| TiO₂ | | 0-1 | | | |
| ZnO | | | | < 0,1 | |
| BaO | | | | <0,1 | |
| F₂ | | | | < 1,0 | |

2. Utilisation selon la revendication 1, **caractérisée en ce que** les paillettes de verre ont été colorées dans la masse à l'aide de colorants inorganiques.

3. Utilisation selon la revendication 1 ou 2, **caractérisée en ce que** les colorants sont les cations ou anions complexes des éléments Cu, Cr, Mn, Fe et Co et/ou des combinaisons de ceux-ci ou TiO₂ ou des métaux nobles élémentaires.

4. Utilisation selon l'une ou plusieurs parmi les revendications 1 à 3, **caractérisée en ce que** les paillettes de verre un rapport d'aspect allant de 1 à 1500.

5. Utilisation selon l'une ou plusieurs parmi les revendications 1 à 4, **caractérisée en ce que** les paillettes de verre possèdent une valeur d'absorption d'huile (déterminée conformément à DIN ISO 787-5: 1995-10) allant de 20 à 130.

6. Utilisation selon l'une ou plusieurs parmi les revendications 1 à 5, **caractérisée en ce que** les paillettes de verre sont amorphes.

7. Utilisation selon l'une ou plusieurs parmi les revendications 1 à 6, **caractérisée en ce que** les paillettes de verre sont combinées dans la formulations avec de l'eau, un alcool, des polyols, des huiles polaires et non polaires, des matières grasses, des cires, des agents filmogènes, des polymères, des copolymères, des agents tensioactifs, des agents anti-radicalaires, des antioxydants, des stabilisants, des exhausteurs d'odeur, des huiles de silicone, des émulsifiants, des fragrances, des solvants, des conservateurs, des épaississants, des additifs rhéologiques et/ou des auxiliaires tensioactifs.

8. Utilisation selon l'une ou plusieurs parmi les revendications 1 à 7, **caractérisée en ce que** les paillettes de verre possèdent une épaisseur de 100 nm - 1 µm.

9. Utilisation selon l'une ou plusieurs parmi les revendications 1 à 8, **caractérisée en ce que** les paillettes de verre possèdent une épaisseur de 600 nm - 1 µm.
